(19) Europäisches Patentamt

European Patent Office

Office européen des brevets

(11) Veröffentlichungsnummer: **0 173 088 B1**

(12) # EUROPÄISCHE PATENTSCHRIFT

(45) Veröffentlichungstag der Patentschrift: **29.04.92**

(21) Anmeldenummer: **85109442.5**

(22) Anmeldetag: **26.07.85**

(51) Int. Cl.⁵: **B01J 35/02**, B01J 23/70,
B01J 23/72, B01J 23/74,
C01C 1/00, C07C 5/00

(54) **Verfahren zur Herstellung von Ammoniak und zur Hydrierung von Olefinen.**

(30) Priorität: **27.07.84 CH 3679/84**

(43) Veröffentlichungstag der Anmeldung:
**05.03.86 Patentblatt 86/10**

(45) Bekanntmachung des Hinweises auf die
Patenterteilung:
**29.04.92 Patentblatt 92/18**

(84) Benannte Vertragsstaaten:
**AT BE CH DE FR GB IT LI LU NL SE**

(56) Entgegenhaltungen:
**DE-A- 2 806 232**
**US-A- 3 876 557**

**Patent Abstracts of Japan, unexamined applications, Sektion C, Band 7, Nr. 281, 15.
Dezember 1983 THE PATENT OFFICE JAPA-
NESE GOVERNMENT Seite 71 C 200 * Kokai-
Nr. 58-159 847 (INOUE)**

(73) Patentinhaber: **LONZA AG**

**Gampel/Wallis(CH)**

(72) Erfinder: **Franzen, Volker, Prof. Dr.**
**C.F. Meyer-Strasse 32**
**Basel(CH)**
Erfinder: **Güntherodt, Hans-Joachim, Prof. Dr.**
**Dorneckweg 10**
**Witterswil Kanton Solothurn(CH)**
Erfinder: **Baiker, Alphons, Dr.**
**Rietgrabenstrasse 63**
**Glattbrugg Kanton Zürich(CH)**
Erfinder: **Armbruster, Erich, Dipl.-Physiker**
**Lindenstrasse 33**
**Allschwil Kanton Baselland(CH)**
Erfinder: **Baris, Halim, Dipl. Chem.-Ing.**
**Wilstrasse 40**
**Dübendorf Kanton Zürich(CH)**

EP 0 173 088 B1

JOURNAL OF CATALYSIS, Band 83, 1983, Academic Press G.V. SMITH et al. "Characterization of Pd-on-Alumina and Pd-Si Glasses by Isomerization and Hydrogenation of ( + )-Apopinene" *Gesamt*

(74) Vertreter: **Schmied-Kowarzik, Volker, Dr. et al Patentanwälte Dr. V. Schmied-Kowarzik Dipl.-Ing. G. Dannenberg Dr. P. Weinhold Dr. D. Gudel Dipl.-Ing. S. Schubert Dr. P. Barz Siegfriedstrasse 8 W-8000 München 40(DE)**

**Beschreibung**

Es ist bekannt, daß bestimmte amorphe Metall-Legierungen Hydrierungsreaktionen, z.B. von Cyclohexenderivaten (G.V. Smith et al, J. of Catalysis 83 (1983) 238) oder Kohlenmonoxid (Fisher-Tropsch-Reaktion) katalysieren (A. Yokoyama et al, Chemistry Letters 1983, 195).

Die katalytische Wirkung soll auf dem amorphen Zustand der Metalle beruhen. Es ist aber auch beschrieben, daß bei dem System $Pd_{80}Si_{20}$ keine signifikanten Unterschiede bezüglich Selektivität bei Hydrierungsreaktionen zwischen amorphem und kristallinem Zustand bestehen (B. Giessen et al, Mater. Res. Soc. Symp. Proc., Band 18, 1982, 255).

Es ist zu beachten, daß in vorliegender Terminologie glasartig erstarrte Metalle die gleiche Bedeutung haben sollen wie amorphe Metalle.

Bei den meisten Untersuchungen sind Oberflächen und Ordnungszustand der aus amorphen Metallen bestehenden Katalysatoren nicht ausreichend untersucht worden, so daß der Vergleich zwischen amorphem und kristallinem System nicht zulässig ist. Es stellte sich beispielsweise heraus, daß sich die katalytische Wirksamkeit aus Unkenntnis der Zusammenhänge zwischen amorphem und kristallinem System nicht ableiten ließ.

Aufgabe vorliegender Erfindung war es, neue Hydrierungsverfahren unter Verwendung von katalytisch wirksamen, glasartig erstarrten Metallen zur Verfügung zu stellen.

Gegenstand der vorliegenden Erfindung ist ein Verfahren zur Herstellung von Ammoniak aus Stickstoff und Wasserstoff unter Verwendung eines Katalysators, das dadurch gekennzeichnet ist, daß ein glasartig erstarrtes Metall, bestehend aus einer Fe-Zr- oder einer Fe-Zr-Mo-Legierung, als Katalysator verwendet wird.

Ein weiterer Gegenstand ist ein Verfahren zur Hydrierung von Olefinen mit Wasserstoff unter Verwendung eines Katalysators, das dadurch gekennzeichnet ist, daß als Katalysator $Cu_{70}Zr_{30}$ als glasartig erstarrtes Metall verwendet wird.

Die in den erfindungsgemäßen Verfahren verwendeten glasartig erstarrten Metalle zeigen eine unerwartete katalytische Wirksamkeit, die möglicherweise dadurch zustande kommt, daß nebeneinander in hochdispersen Formen amorphe und kristalline Bezirke vorliegen. Es ist ferner beachtlich, daß die Zusammensetzung der Oberfläche vielfach nicht die gleiche ist, wie die der Masse.

Die erfindungsgemäß verwendeten Katalysatoren lassen sich ausgehend von amorphen Legierungen herstellen, die u.a. den Vorteil haben, daß die Metalle in diesem Zustand außerordentlich hochdispers verteilt sind; es können Aggregationen von nur wenigen Atomen sein. Von den bisher benutzten Verfahren zur Katalysatorherstellung führt keines der industriell genutzten Verfahren zu ähnlich hohem Dispersionsgrad.

Bei den erfindungsgemäß verwendeten katalytisch wirksamen, glasartig erstarrten Metallen handelt es sich entweder um Fe-Zr-, Fe-Zr-Mo- oder Cu-Zr-Legierungen.

Vorzugsweise bestehen sie aus Metallen mit der Formel $Fe_{91}Zr_9$, der Formel $Fe_{24}Zr_{76}$ oder der Formel $Cu_{70}Zr_{30}$.

Die erfindungsgemäßen glasartig erstarrten Metalle sind amorphe Metall-Legierungen mit ungeordneter Struktur, die sich nicht im thermodynamischen Gleichgewicht befinden. Glasartig erstarrte Metalle neigen zur Rekristallisation, wenn die Reaktionstemperatur der katalytischen Umsetzung über der Glasumwandlungstemperatur liegt. Durch Legierungsbestandteile bis zu 5 Atom-%, z.B. Molybdän, läßt sich aber die Glasumwandlungstemperatur so weit heraufsetzen, daß eine Stabilisierung des eigentlichen Katalysators erreicht wird, ohne daß dessen Aktivität allzusehr beeinflußt wird.

Stabilisierte glasartig erstarrte Metalle können beispielsweise aus Zr, Fe und Mo bestehen, vorzugsweise mit der Formel $(Fe_{91}Zr_9)_{95}-Mo_5$.

Die katalytisch wirksamen, glasartig erstarrten Metalle können als solche als Katalysatoren eingesetzt werden, da sie sich vielfach selbst aktivieren, teilweise unter Vergrößerung der Oberfläche; ein Beispiel eines derartigen Metalles weist die Formel $Fe_{91}Zr_9$ auf.

Die aus den glasartig erstarrten Metallen hergestellten Katalysatoren eignen sich erfindungsgemäß für den Einsatz in Hydrierungsverfahren, beispielsweise für die Synthese von Ammoniak aus Wasserstoff und Stickstoff und für die Synthese von Kohlenwasserstoffen aus Olefinen.

Die glasartig erstarrten, also amorphen Metalle sind auch als Ausgangsprodukte für Katalysatoren ohne Träger geeignet. Man kann beispielsweise eine der Phasen durch chemische Umsetzung derart umwandeln, daß sie wie ein Träger im konventionellen Sinne wirkt. Als möglicher Nachteil ist ebenfalls eine geringere spezifische Oberfläche, die aus den verschiedenen Herstellungsverfahren resultiert, zu nennen.

Die glasartig erstarrten Metalle können auf an sich bekannte Weise hergestellt werden. Beispielsweise mit dem Schmelz-Spinn-Verfahren, als flache oder getrennte Blättchen und nach dem Splat-Cooling-Verfahren (vergl. z.B. H.-J. Güntherodt und H. Beck "Topics in Applied Physics", Band 46: Glassy Metals I; Nachdruck: Springer-Verlag Berlin Heidelberg, 1981, S. 1-18).

Es hat sich aber gezeigt, daß die nach dem Schmelz-Spinn-Verfahren erhaltenen Bänder auch leicht bei tiefer Temperatur zu Pulver zerkleinert werden und auch in pulveriger Form eingesetzt werden können. Amorphe Metalle lassen sich aber auch direkt als Pulver herstellen. Aus Bändern oder Folien von amorphen Metallen können auch Formkörper, z.B. Kolonnenfüllkörper, hergestellt werden, die dann zum Katalysator aktiviert und als solche eingesetzt werden.

Im Falle von $Cu_{70}Zr_{30}$ zeigen Katalysatoren aus amorphen Metallen Aktivität bereits bei tieferen Temperaturen als die entsprechenden Katalysatoren basierend auf kristallinem Ausgangsmaterial. Wichtig ist, daß die Reaktionstemperatur ausreichend tiefer als die Glasumwandlungstemperatur des metallischen Glases ist.

Die glasartig erstarrten Metalle der Formel $Cu_{70}Zr_{30}$ können im Wasserstoffstrom aktiviert werden und eignen sich dann als Hydrierkatalysator, z.B. zur Hydrierung von 1,3-Butadien, Ethylen oder Gemischen dieser Olefine. Für diese Reaktion ist es vorteilhaft, das glasartig erstarrte Metall mit der Formel $Cu_{70}Zr_{30}$ während etwa 2 bis 8 Stunden bei 160 bis 240°C im Wasserstoffstrom zu reduzieren. Während der Hydrierung soll ein Verhältnis von Olefin zu Wasserstoff von 2:1 bis 1:1 und eine Temperatur von 75 bis 200°C, vorzugsweise 95 bis 130°C eingehalten werden.

Eine Katalysatoraktivierung kann jedoch auch durch eine Behandlung mit Säuren, zweckmäßig verdünnten Säuren und $HNO_3$, vorzugsweise wässriger $HNO_3$, durch die die Oxidschichten entfernt werden, und anschließender Behandlung mit Sauerstoff und nachfolgend mit Wasserstoff erfolgen.

Die Aktivierung besteht demnach in einer Behandlung in oxidierender Atmosphäre und darauffolgender Behandlung in reduzierender Atmosphäre.

Beispiel 1

Synthese von Ammoniak aus Stickstoff und Wasserstoff.

Für die Umsetzung wurde eine Gasumsetzung von 75% Wasserstoff und 25% Stickstoff eingesetzt. Das Gasgemisch war frei von Kohlenmonoxid. Der Druck betrug 9 bar. In einen mikrokontinuierlichen Reaktor wurden 2 g Katalysator eingefüllt, die Länge des Katalysatorbettes betrug 20 mm, die Durchflußmenge lag zwischen 20 und 200 Mikromol.sec$^{-1}$.

| Ausgangsmaterial | Gleichgewichtsumsatz Nanomol.sec$^{-1}$ | | | Bemerkung |
|---|---|---|---|---|
| | 380°C | 400°C | 420°C | |
| Konventioneller Halder-Topsöe-Katalysator | 250 | 450 | 700 | nach 2000h nicht stabil |
| $Fe_{91}Zr_9$-Glas | 800 | 1400 | 2000 | stabil nach 2000 h |
| $Fe_{91}Zr_9$-kristallin | 140 | 260 | 400 | stabil nach 2000 h |
| $(Fe_{91}Zr_9)_{95}$-$Mo_5$-Glas | 180 | 330 | 500 | stabil |

Unter Gleichgewichtsumsatz ist der auf die Oberfläche der Ausgangsmaterialien normierte Umsatz pro Kontaktzeit (= durchschnittliche Aufenthaltszeit eines Gasmoleküls im Kontaktvolumen) angegeben. Der Halder-Topsöe-Katalysator ist gegenüber Sauerstoff empfindlicher als das $Fe_{91}Zr_9$-Glas.

Beispiel 2

Synthese von Ammoniak.

Die Umsetzung wurde in einem Integral-Reaktor aus rostfreiem Stahl (40 cm Länge, 1,5 cm Durchmesser) mit gereinigten Gasen durchgeführt. Analyse der Reaktionsprodukte mittels IR-Gasanalysator. Der Druck betrug 4 bar, der Totalgasdurchfluß 30 bis 40 $ml_N.min^{-1}$ Katalysatormenge 8 bis 10 g. Die Bänder aus amorphem Metall wurden entfettet und in Stücke von 1 bis 2 cm Länge zerschnitten.

| Ausgangsmaterial | Temp. °C | $Umsetzungsgrad\,\eta = \dfrac{[NH_3]}{[NH_3]eq}$ |
|---|---|---|
| $Fe_{91}Zr_9$ amorph | 350 | 0.001704 |
| $Fe_{91}Zr_9$ krist. | 350 | 0.001309 |
| Fe rein krist. | 380 | 0.000144 |
| $Fe_{91}Zr_9$ amorph | 380 | 0.005089 |
| $Fe_{91}Zr_9$ krist. | 380 | 0.004801 |
| Fe rein krist. | 450 | 0.00835 |
| $Fe_{91}Zr_9$ krist. | 450 | 0.03268 |
| $Fe_{24}Zr_{76}$ amorph | 450 | 0.08170 |
| $Fe_{24}Zr_{76}$ amorph | 380 | 0.002880 |

$$Verhältnis\ N_2:H_2\ =\ 1:2$$

Daß zwischen den Metallen in dem eigentlich wirksamen Katalysator Wechselwirkungen bestehen, zeigt sich im Vergleich der Umsatzzahlen für die Ammoniaksynthese beim System Eisen-Zirkon. Während reines Eisen bei 35o°C keinen aktiven Katalysator ergibt, bilden $Fe_9Zr_{91}$- und $Fe_{24}Zr_{76}$-Gläser aktive Katalysatoren. Während bei 400°C $Fe_{91}Zr_9$ aktiver als $Fe_{24}Zr_{76}$ ist, übertrifft bei höheren Temperaturen $Fe_{24}Zr_{76}$ die Aktivität von $Fe_{91}Zr_9$. Die Möglichkeiten über amorphe Metalle hochaktive katalytische Systeme zu erhalten, sind sehr vielfältig.

Beispiel 3

Hydrierung von Ethylen.

Die Untersuchung wurde in einem Kreislaufreaktor durchgeführt, die Produkte mittels Gaschromatographie analysiert. Die amorphen Metalle wurden als Streifen von etwa 1 cm Länge eingesetzt, nachdem sie entfettet waren. Das Reaktionsgemisch bestand aus Ethylen und Wasserstoff.

$Fe_{91}Zr_9$-Glas, das erst mit verdünnter Salpetersäure, dann mit Sauerstoff und nachfolgend mit Wasserstoff behandelt wurde, zeigte nach einer solchen Vorbehandlung keine Aktivität. $Cu_{70}Zr_{30}$-Glas zeigte bei der Behandlung mit Wasserstoff eine deutliche Vergrößerung der Oberfläche und hervorragende katalytische Aktivität.

| Katalysator | Reduktion | Aktivität |
|---|---|---|
| $Cu_{70}Zr_{30}$ amorph | 200°C $H_2$ 4 h | sehr aktiv auch bei 80°C |
| $Cu_{70}Zr_{30}$ krist. | 200°C $H_2$ 4 h $H_2$ 8 h | --- --- |
| Cu | 200°C $H_2$ 4 h | --- |

Mit amorphem $Cu_{70}Zr_{30}$ wurde nach Aktivierung bei 200°C in 24 min. parallel quantitativer Umsatz gemessen. In der gleichen Zeit betrug der Umsatz bei 80°C bereits 40%.

Der Unterschied zwischen amorphem und kristallinem Ausgangsmaterial zeigte sich ganz besonders deutlich bei der Hydrierung von Ethylen mittels $Cu_{70}Zr_{30}$. Nur das amorphe Ausgangsmaterial ergab einen aktiven Katalysator.

Beispiel 4

Hydrierung von 1,3-Butadien.

Die Reaktionen wurden in einem Batch-Kreislaufreaktor durchgeführt und die Produkte, bestehend aus 1-Buten, cis-2-Buten, trans-2-Buten und Butan, wurden mittels Gaschromatographie analysiert.

Amorphe und kristalline Proben der Zusammensetzung $Cu_{70}Zr_{30}$ wurden bei 200°C 4 Std. lang im Wasserstoffstrom reduziert. Diese Vorbehandlung verursachte bei der amorphen Probe eine Vergrößerung der Oberfläche von 0,015 $m^2$/g auf 0,56 $m^2$/g, während die Oberfläche der kristallinen Probe unverändert bei 0,008 $m^2$/g blieb.

Um die Aktivität dieser Proben vergleichen zu können, wurden Katalysatormengen so gewählt, daß im Reaktor gleich große Oberflächen vorhanden waren. Diese Versuche unter identischen Versuchsbedingungen (T = 130°C, p = 0,8 bar, Butadien:$H_2$ = 1:1) zeigen deutlich, daß amorphes $Cu_{70}Zr_{30}$ viel aktiver als die entsprechende kristalline Probe war.

| t (min) | 0,12 g Amorph Umsatz % | 8,0 g Kristallin Umsatz % |
|---|---|---|
| 25 | 4,59 | 0,0 |
| 70 | 13,04 | 0,0 |
| 90 | 16,76 | 0,0 |
| 130 | 23,00 | 0,9 |
| 130 | 26,00 | 1,1 |

Mit der amorphen Probe wurde die Selektivität bezüglich Buten näher untersucht Bei 90% Umsatz betrug die Selektivität bei 130°C 75% und hei 95°C 96%.

Beispiel 5

Selektive Hydrierung von Butadien.

Diene, besonders 1,3-Butadien, verursachen Desaktivierung des Katalysators bei der Hydroformylierung und bilden Polymere bei Crackoperationen. Deshalb sollen sie von den Olefinen entfernt werden.

4 g amorphes $Cu_{70}Zr_{30}$ wurde gemäß Beispiel 4 als Katalysator eingesetzt. Die Hydrierung des Gemisches mit der Zusammensetzung: 73% 1-Buten, 24% cis-2-Buten und 3% 1,3-Butadien wurde bei verschiedenen Temperaturen untersucht. Bei Temperaturen höher als 90°C wurden auch Olefine hydriert, und es entstand eine große Menge von Butan. Bei 75°C wurde Butadien selektiv hydriert, und die Produktverteilung nach einer Reaktionszeit von 80 Min. besteht aus: 1,63% Butan, 1,35% trans-2-Buten, 22,6% cis-2-Buten, 74,41% 1-Buten und 0,0% Butadien.

Die Wasserstoffkonzentration war dabei 2-4 mal größer als die Butadienkonzentration. In diesem Bereich wies die Wasserstoffkonzentration keinen großen Einfluß auf die Selektivität auf. Die selektive Hydrierung von Butadien im Gemisch von Ethylen und Butadien erfolgte auch bei tieferen Temperaturen. Die Reaktionstemperatur von 75°C ermöglichte die Hydrierung von Butadien mit 93% Selektivität an Butenen; Ethylen wurde dabei gar nicht hydriert. Höhere Temperaturen verursachten jedoch auch die Hydrierung von Ethylen.

**Patentansprüche**

1. Verfahren zur Herstellung von Ammoniak aus Stickstoff und Wasserstoff unter Verwendung eines Katalysators, dadurch gekennzeichnet, daß ein glasartig erstartes Metall, bestehend aus einer Fe-Zr- oder einer Fe-Zr-Mo-Legierung, als Katalysator verwendet wird.

2. Verfahren nach Patentanspruch 1, dadurch gekennzeichnet, daß als glasartig erstarrtes Metall $(Fe_{91}Zr_9)_{95}Mo_5$ verwendet wird.

3. Verfahren nach Patentanspruch 1, dadurch gekennzeichnet, daß $Fe_{24}Zr_{76}$ verwendet wird.

4. Verfahren nach Patentanspruch 1, dadurch gekennzeichnet, daß $Fe_{91}Zr_9$ verwendet wird.

5. Verfahren nach einem der Patentansprüche 1 bis 4, dadurch gekennzeichnet, daß die Umsetzung in einem Temperaturbereich von 350 bis 420°C erfolgt.

6. Verfahren nach einem der Patentansprüche 1 bis 5, dadurch gekennzeichnet, daß das glasartig erstarrte Metall durch Selbstaktivierung aktiviert worden ist.

7. Verfahren zur Hydrierung von Olefinen mit Wasserstoff unter Verwendung eines Katalysators, dadurch gekennzeichnet, daß als Katalysator $Cu_{70}Zr_{30}$ als glasartig erstarrtes Metall verwendet wird.

8. Verfahren nach Patentanspruch 7, dadurch gekennzeichnet, daß als Olefin 1,3-Butadien, Ethylen oder ein Gemisch der genannten Olefine eingesetzt wird.

9. Verfahren nach einem der Patentansprüche 7 oder 8, dadurch gekennzeichnet, daß die Hydrierung in einem Temperaturbereich von 75 bis 200°C erfolgt.

10. Verfahren nach einem der Patentansprüche 7 bis 9, dadurch gekennzeichnet, daß das Verhältnis Olefin zu Wasserstoff zwischen 2:1 und 1:1 liegt.

**Claims**

1. Process for the preparation of ammonia from nitrogen and hydrogen using a catalyst, characterized in that a vitrified metal consisting of a Fe-Zr- or a Fe-Zr-Mo-alloy is used as catalyst.

2. Process according to patent claim 1, characterized in that $(Fe_{91}Zr_9)_{95}Mo_5$ is used as vitrified metal.

3. Process according to patent claim 1, characterized in that $Fe_{24}Zr_{76}$ is used.

4. Process according to patent claim 1, characterized in that $Fe_{91}Zr_9$ is used.

5. Process according to any one of patent claims 1 to 4, characterized in that the reaction is carried out at a temperature range of 350 to 420°C.

6. Process according to any one of patent claims 1 to 5, characterized in that the vitrified metal has been activated by autoactivation.

7. Process for the hydrogenation of olefins with hydrogen using a catalyst, characterized in that as catalyst $Cu_{70}Zr_{30}$ is used as vitrified metal.

**8.** Process according to patent claim 7, characterized that as olefin 1,3-butadiene, ethylene or a mixture of these olefins is used.

**9.** Process according to any one of patent claims 7 to 8, characterized in that the hydrogenation is carried out at a temperature range of 75 to 200°C.

**10.** Process according to any one of patent claims 7 to 9, characterized in that the ratio of olefin to hydrogen is between 2:1 and 1:1.

**Revendications**

**1.** Procédé de préparation d'ammoniac à partir d'azote et d'hydrogène à l'aide d'un catalyseur, caractérisé en ce que l'on utilise comme catalyseur un métal vitreux consistant en un alliage Fe-Zr ou Fe-Zr-Mo.

**2.** Procédé selon la revendication 1, caractérisé en ce que l'on utilise $(Fe_{91}Zr_9)_{95}Mo_5$ comme métal vitreux.

**3.** Procédé selon la revendication 1, caractérisé en ce que l'on utilise $Fe_{24}Zr_{76}$.

**4.** Procédé selon la revendication 1, caractérisé en ce que l'on utilise $Fe_{91}Zr_9$.

**5.** Procédé selon l'une des revendications 1 à 4, caractérisé en ce que la réaction a lieu dans un domaine de température de 350 à 420°C.

**6.** Procédé selon l'une des revendications 1 à 5, caractérisé en ce que le métal vitreux a été activé par autoactivation.

**7.** Procédé d'hydrogénation d'oléfines avec l'hydrogène à l'aide d'un catalyseur, caractérisé en ce que l'on utilise comme catalyseur $Cu_{70}Zr_{30}$ comme métal vitreux.

**8.** Procédé selon la revendication 7, caractérisé en ce que l'on utilise comme oléfine le butadiène-1,3, l'éthylène ou un mélange des oléfines citées.

**9.** Procédé selon l'une des revendications 7 ou 8, caractérisé en ce que l'hydrogénation a lieu dans un domaine de température de 75 à 200°C.

**10.** Procédé selon l'une des revendications 7 à 9, caractérisé en ce que le rapport oléfine à hydrogène est compris entre 2:1 et 1:1.